# EUROPEAN PATENT SPECIFICATION

(11) **EP 3 706 671 B1**
(45) Date of publication and mention of the grant of the patent: **05.01.2022**
(21) Application number: 18799663.2
(22) Date of filing: 02.11.2018
(51) Int. Cl.: A61F 5/44, A61F 5/445

(54) **COMPLEMENTARY MATERIAL DISPENSER IN A BODY WASTE COLLECTING APPLIANCE AND AN OSTOMY APPLIANCE SYSTEM**
KOMPLEMENTÄRMATERIALSPENDER IN EINER VORRICHTUNG ZUM AUFFANGEN VON KÖRPERSCHLACKEN UND OSTOMIEVORRICHTUNGSYSTEM
DISTRIBUTEUR DU MATERIEL COMPLEMENTAIRE DANS UN DISPOSITIF COLLECTEUR DE DÉCHETS CORPORELS ET SYSTEME D'APPAREIL D'OSTOMIE

(30) Priority: 08.11.2017 DK PA201770839
(43) Date of publication of application: 16.09.2020
(73) Proprietor: Coloplast A/S, 3050 Humlebaek (DK)
(72) Inventor: MORGAN HICKMOTT, Richard, 3000 Helsingoer (DK)
(86) International application number: PCT/DK2018/050281
(87) International publication number: WO 2019/091529

(56) References cited:
- WO-A1-98/01094
- GB-A- 2 351 442
- US-A- 3 690 320
- US-B1- 6 656 169

## Description

### Background

Stomal output often contains body fluids and visceral contents that are aggressive to both the skin of a user and to ostomy devices, in particular these have a detrimental effect on the efficiency and integrity of the adhesive materials that are applied to attach the ostomy device to the user's skin surface. Some ostomists may choose or have to wear their device for prolonged periods of time. For users in general, and particularly for these ostomists safe, reliable and efficient ostomy devices are highly desirable. Numerous attempts have been made to provide ostomy devices to meet such demands, e.g. the demand of prolonged wear time, but the provision of sufficient efficiency to achieve a satisfactory long wear time of ostomy devices continues to be an unmet need.

WO98/01094 discloses an excrement collecting bag having a bag body detachably mounted around an ostomy of a user, which is provided with a deodorant sealing portion. When stomal output is removed from the bag, the deodorant sealing portion is broken to permit a deodorant to be discharged into the bag body to prevent generation of foul odours.

Ostomists and health care professionals alike would welcome improvements in ostomy devices to better meet such demands.

### Summary

The present disclosure provides aspects of an ostomy appliance system including a complementary material dispenser attachable to an interior surface of a top portion of a collecting bag for body waste according to the appended claims.

### Brief Description of the Drawings

The accompanying drawings are included to provide a further understanding of embodiments and are incorporated in and constitute a part of this specification. The drawings illustrate embodiments and together with the description explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Figure 1 is a schematic, perspective view of one embodiment of a complementary material dispenser and further illustrates an ostomy appliance system including the complementary material dispenser.
Figure 2 is a schematic cross-sectional view of one embodiment of a complementary material dispenser provided inside the collecting bag of an ostomy appliance and illustrating the release of complementary material from the complementary material dispenser.
Figure 3 is a schematic perspective view of one embodiment of a complementary material dispenser.
Figure 4 is a schematic perspective view of one embodiment of a complementary material dispenser.

### Detailed Description

In the following Detailed Description, reference is made to the accompanying drawings, which form a part hereof, and in which is shown by way of illustration specific embodiments in which the invention may be practiced. In this regard, directional terminology, such as "top", "bottom", "front", "back", "leading", "trailing" etc., is used with reference to the orientation of the Figure(s) being described. Because components of embodiments can be positioned in a number of different orientations, the directional terminology is used for purposes of illustration and is in no way limiting. It is to be understood that other embodiments may be utilized and structural or logical changes may be made without departing from the scope of the present invention. The following detailed description, therefore, is not to be taken in a limiting sense, and the scope of the present invention is defined by the appended claims.

It is to be understood that the features of the various exemplary embodiments described herein may be combined with each other, unless specifically noted otherwise.

Throughout this disclosure, the words "stoma" and "ostomy" are used to denote a surgically created opening bypassing the intestines or urinary tract system of a person. The words are used interchangeably, and no differentiated meaning is intended. The same applies for any words or phrases derived from these, e.g. "stomal", "ostomies" etc. Also, the solid and liquid wastes emanating from the stoma may be referred to as both stomal "output", "waste(s)", and "fluids" interchangeably.

By body waste or (stomal) output is herein meant the effluent from a stoma, being faeces and/or urine in a more or less viscous form and/or mucins secreted from the epithelial layer of the alimentary canal. In the case of a colostomy, the output may be quite solid, whereas an ileostomy may produce more liquid output. The body waste or output may contain digestive fluids with enzymes and other components that may be aggressive to the skin and thus may cause skin problems including damage, maceration and contact dermatitis of the skin, if brought into contact with it as well as the output may comprise components that may attack and degrade the adhesive.

A person having undergone ostomy surgery may be referred to as "ostomist" or "ostomate" - moreover, also as "patient" or "user". However, in some cases "user" may also relate or refer to a health care professional (HCP), such as a surgeon or an ostomy care nurse or others. In those cases, it will either be explicitly stated, or be implicit from the context that the "user" is not the "patient" him- or herself.

In the following, whenever referring to proximal side of a device or part of a device, the referral is to the skin-facing side, when the appliance is worn by a user. Likewise, whenever referring to the distal side of a device or part of a device, the referral is to the side facing away from the skin, when the appliance is worn by a user. In other words, the proximal side is the side closest to the user, when the appliance is fitted on a user and the distal side is the opposite side - the side furthest away from the user in use.

The axial direction is defined as the direction of the stoma, when the appliance is worn by a user. Thus, the axial direction is generally perpendicular to the skin or abdominal surface of the user. To some extent the 'generally perpendicular direction of the stoma' may be perceived more as an ideal rather than real direction, since many factors and dynamics continuously influence a stoma's condition.

The radial direction is defined as transverse to the axial direction that is transversely to the direction of the stoma. In some sentences, the words "inner" and "outer" may be used. These qualifiers should generally be perceived with reference to the radial direction, such that a reference to an "outer" element means that the element is farther away from a centre portion of the ostomy appliance than an element referenced as "inner". In addition, "innermost" should be interpreted as the portion of a component forming a centre of the component and/or being adjacent to the centre of the component. In analogy, "outermost" should be interpreted as a portion of a component forming an outer edge or outer contour of a component and/or being adjacent to that outer edge or outer contour.

Appliances for collecting body wastes in a collecting bag, such as an ostomy appliance are widely available. The collecting bag usually comprises a front wall on the distal side and a rear wall on the proximal side. The walls are made of gas- and liquid impermeable foil-material (for example of polyethylene (PE), polyvinyl-chloride (PVC) or ethylene-vinylacetate (EVA)) that is welded around the edges or the rim to form a pouch defining a body waste collecting volume or chamber. The collecting bag may be welded only partly around the rim so that a discharge opening for emptying the collecting bag is provided at the bottom of the bag. In that case the collecting bag can include means allowing for repeated opening and closing of the discharge opening. A body waste inlet opening is provided in the rear wall and is regularly placed in the upper part of the collecting bag so that when a user wearing the appliance stands up, the body waste inlet opening locates above the midline of the collecting bag. This means that a larger collecting volume is located below the body waste inlet opening. Thus, the top portion of the collecting bag is defined as the part of the bag closest to the body waste inlet opening, and the bottom portion is defined as the opposite part, farthest away from the body waste inlet opening.

The use of the phrase "substantially" as a qualifier to certain features or effects in this disclosure is intended to simply mean that any deviations are within tolerances that would normally be expected by the skilled person in the relevant field.

The use of the phrase 'complementary material' is intended to mean that the dispenser dispenses material, which is a 'surplus', an 'extra' or an 'additional' (i.e. complementary) material component in relation to another material of a body waste collecting appliance, such as, but not limited to, the adhesive material on a base plate of an ostomy appliance. The complementary material can include one or more releasable substances, such as, but not limited to body waste neutralizers or neutralizing components or compositions.

In the context of the present disclosure, a "pocket" should be considered a compartment or cavity, i.e. a structure forming, or allowing for, the presence of a volume or space that is surrounded by something and allowing for temporarily or permanently holding something else in the volume or space. In a non-limiting example, the pocket is provided on or in the complementary material dispenser and is configured to allow the user to insert one or more fingers therein for temporarily moving the complementary material dispenser into a desired location.

When a body waste collecting device is applied to the skin surface of a user, in particular when an ostomy appliance is applied to the skin surrounding a stoma, adhesive on a base plate of the appliance ideally provides a tight fit or sealing to the skin, to avoid stomal output from propagating under the adhesive of the base plate, which is potentially damaging to the skin and prone to degrading the adhesive as explained above. Any stomal output propagating or seeping under the adhesive of base plate is to be avoided as best as possible because contents of such output can lead to damage and/or maceration of the skin and degradation of the adhesive, potentially resulting in adhesive failure and eventually leakage of the stomal output onto the user's clothes, presenting a stigmatising embarrassment and clearly discomfort to the user. However, experience has shown that avoiding any or all kind of such problems is extremely difficult and that users regularly are faced with these issues.

According the disclosure, such adhesive failures and other problems can be overcome or at least significantly reduced in that a complementary material is dispensable from a complementary material dispenser attached to an interior surface of a top portion of the collecting bag. Particularly, but not exclusively, a neutralizing composition provided in the complementary material dispenser can be configured to be released in the presence of moisture and thereby act to neutralize the aggressive contents of the body waste or output to help avoid or reduce damage and/or maceration of the skin and/or degradation of the adhesive. Additionally, and/or alternatively, the neutralizer or neutralizing composition acts to slow or decelerate the damaging effects of the body waste or output, in some cases providing for increased or prolonged wear time of the waste collecting appliance, which in turn provides both practical and economic advantages.

When preparing a base plate of an ostomy appliance to fit an individual user's stoma and body (skin surface), it often involves trimming or cutting (e.g. with special scissors) into correct shape and size an ostomy receiving opening in the base plate of the appliance. To avoid the thereby cut edge from gnawing on the stoma, and since adaptation of such opening in the base plate is difficult to make with a great deal of precision, the cutting/adaptation almost always leaves a small gap (distance) between an edge of the cut opening in the adhesive base plate and the user's stoma, when the appliance is applied around the stoma of the user. However, such gap does help to provide room for the stoma to 'work' (i.e. expand and contract), caused *inter alia* by peristaltic movements of the intestine; often, the stoma enlarges when delivering stomal output and shortens when not. This gap is also called the peristomal gap. Stomal output inevitably flows into the peristomal gap and will over time be able to propagate under the adhesive interface between the skin and the base plate from the peristomal gap. Consequently, the necessary provision of an ostomy receiving opening and/or the peristomal gap also contributes to the risk of damage and degradation of the adhesive layer of the base plate followed potentially by skin irritation, damage, maceration and/or other skin problems.

By providing a complementary material dispenser comprising a neutralizer or neutralizing component together with a base plate for an ostomy appliance, the dispenser provides for the neutralizing component to be directed into contact with the stomal output and into the peristomal gap. Both the user's skin and the adhesive layer on the base plate at, and adjacent to the peristomal gap, will thereby be protected from the damaging effects of the stomal output by the released neutralizer. The dispenser of this disclosure is configured to provide for the neutralizer to be released and directed towards a material allocation zone including at a top portion of the collecting bag inside the bag, such as for example to ensure that a major portion of the neutralizing component is directed to flow into the peristomal gap and/or onto a distal surface of the base plate of the ostomy appliance to interact with stomal output to neutralize its harmful components and avoid or delay degradation of the adhesive layer of the base plate.

Experience shows that stomal output can flow substantially continuously or enter the collecting bag in bursts when exiting the stoma, depending among other factors on the type of stoma. If a user of an ostomy appliance is in an upright position, stomal output may flow continuously downwards due to gravity and thereby act to primarily 'wet' or 'soil' a portion of the base plate located below (beneath or under) the stoma when the appliance is worn. However, stomal output is known to also creep upwards to 'wet' or 'soil' a portion of the base plate located above (higher or over) the stoma when the appliance is worn.

Particularly, when stomal output enters the inside of a collecting bag in bursts, with a distal wall of the collecting bag being pressed close to the stoma (e.g. due to the user's clothing, belt, trouser lining etc.), the stomal output is known to be able to spread all over a central portion of the base plate, including also the area above (higher than/over) the stoma. Thus, stomal output may not immediately or exclusively be forced *downwards* by gravity, but can also be affected (by force(s) acting on it) to 'wet' or 'soil' an area of the collecting bag above (over) an inlet opening in the collecting bag and possibly also 'soil' the peristomal gap.

In conclusion, during normal use of an ostomy appliance, stomal output does not subject itself to an 'idealistic behaviour' of simply being forced by gravity to the 'bottom' of the collecting bag's reservoir volume. Instead, stomal output can (or be forced to) travel in any direction from the inlet opening of the collecting bag, including to areas/locations above (higher/over) the stoma. In some cases, stomal output can even be smeared against the walls (distal and/or proximal) of the collecting bag and further onto anywhere on a distal surface of the base plate and 'back' into the peristomal gap. Consequently, it is not easy to foresee exactly where and how fast stomal output will 'end up' and present a problem. The present disclosure provides solutions which help avoid or prevent adhesive failure or at least reduce the impact of the stomal output's potentially damaging and detrimental effects on the user's skin surface, stoma and/or on the components of the ostomy appliance by offering a complementary material dispenser which is configured to release the complementary material, such as a 'mitigating' neutralizer or neutralizing component.

From the above, it is understood that in conceiving the invention of the present disclosure, the inventors realized that the complementary material and/or the neutralizing component must not *per se* be provided close to, or in direct contact with, the stoma's surface, or directly onto the peristomal gap or skin surface for the complementary material and/or the neutralizing component to be able to provide its beneficial effects. Indeed, it was realized that the material's effects are achievable *inter alia* by allowing the complementary material and/or the neutralizing component to be released at or into the peristomal gap; into an area of the collecting bag around the bag's inlet opening, and/or on the base plate, such as on a central portion of the base plate, including on a distal surface of the base plate facing away from the skin of the user, when the ostomy appliance is worn. Moreover, it is further realized that it is additionally or alternatively beneficial to have the complementary material and/or the neutralizing component released into a portion of the collecting bag, particularly and especially released towards a material allocation zone at a top portion of the collecting bag inside the bag. In some implementations, the complementary material dispenser can be adapted to release the complementary material and/or the neutralizing component in more than one direction, such as towards a proximal surface of the base plate (facing the user's skin) and a distal surface of the base plate (facing or being exposed to the inside of the collecting volume of the collecting bag).

In one **aspect**, the present disclosure relates to an ostomy appliance system including a complementary material dispenser configured to be attachable to an interior surface of a top portion of a collecting bag for body waste, and to provide dispensing of complementary material from the dispenser towards a material allocation zone at the top portion of the collecting bag.

The material allocation zone at the top portion of the collecting bag can be understood as a zone or location wherein delivery of the complementary material is particularly beneficial, because that zone or location is exceedingly prone to being contaminated or 'attacked' by body waste, such as stomal output.

Particularly, but not exclusively, the material allocation zone can be defined as being at one or more of the following zones or locations: at and/or in the body waste inlet opening; at the interior surface of a proximal wall of the collecting bag adjacent to and/or surrounding the body waste inlet opening, and/or at the interior surface of a distal wall of the collecting bag across from the body waste inlet opening. Moreover, configuring the dispenser to direct complementary material to one or more of these zones or locations helps provide for the complementary material to enter through (out of/via) the body waste inlet opening of the collecting bag and into the peristomal skin surface area, and/or into the peristomal gap between the stoma and an inner periphery of a receiving opening in the base plate of the body waste collecting appliance.

It is understood that the zones or locations which can define a material allocation zone in each case are present at the top portion of the collecting bag. In this disclosure, it is foreseen that attachment of the complementary material dispenser to an interior surface of a top portion of a collecting bag for body waste is particularly advantageous, *inter alia* in that it enables delivery of complementary material at a location of the waste collecting appliance where the major portion of and the most 'aggressive' body waste or output passes the base plate and enters into the collecting bag of the appliance. Furthermore, dispensing the complementary material towards a material allocation zone at the top portion of the collecting bag provides for additional protection of the adhesive of the base plate, and also of the skin surface of the user, because the complementary material being dispensed can engage with the body waste as soon as the waste exudes or leaves the body waste opening, such as a stoma. Thereby, none or at least a lesser portion of the exuding body waste or output can 'attack' the adhesive material and the skin surface.

The complementary material dispenser can be provided by a variety of different structures or in different implementations. It is to be understood that the complementary material dispenser of the disclosure is intended as and configured to be a single-use component or element that is to be frequently substituted for a new, such as, but not exclusively, at the times when the user changes the waste collecting appliance.

In embodiments, the complementary material dispenser comprises a receptacle for housing the complementary material. In embodiments, the complementary material dispenser comprises a receptacle for housing a releasable complementary material composition. In a variety of embodiments, the receptacle can be configured to be open, closed or partially opened. In embodiments, the receptacle is configured to be openable, e.g. by comprising one or more walls comprising a material soluble by contact with moisture. In embodiments, one suitable material for the one or more walls of a moisture-soluble film is a PVOH based thermoplastic film, such as a Monosol^{®} 7031 film from kurakay WS Film Division^{™}, Portage, Indiana, United States.

In embodiments, the receptacle can include any one of known types of receptacles, such as, but not limited to pouches, a bag, 'tea bag', envelope, capsule, packet, sachet, or container. In one embodiment, the receptacle can be understood as being similar to a tea bag.

In embodiments, the receptacle includes at least one outlet for dispensing the complementary material from the dispenser. In embodiments, the receptacle includes a single (one and only one) outlet. In embodiments, at least one outlet of the receptacle is provided in a first end portion of the receptacle, the first end portion configured to be located closest to a bottom of a collecting bag, when the dispenser is attached to the interior surface of a top portion of the collecting bag. In embodiments, the receptacle includes two major and two minor external surfaces wherein at least one outlet is provided in each of the minor external surfaces. In embodiments, the two major external surfaces of the receptacle are configured to extend substantially in parallel with the proximal wall and the distal wall of the collecting bag, and the two minor external surfaces combine with the major external surfaces to form sides of the receptacle connecting the two major external surfaces. In embodiments, the receptacle includes a plurality of outlets distributed in a pattern over an external surface of the receptacle.

The number and specific distribution of the one or more outlets of the receptacle can be configured to help provide and control the delivering of complementary material releasable from the dispenser towards at least one material allocation zone at the top portion of the collecting bag, such as by directing the complementary material towards the peristomal area or into the peristomal gap of an ostomy appliance.

In embodiments, the complementary material dispenser comprises a foam element. In embodiments, one or more complementary material compositions is/are contained in the foam element. In embodiments, the foam element is infused with one or more complementary material compositions. In embodiments, the complementary material includes at least one neutralizer or neutralizing component contained in the foam element. In embodiments, the foam element is infused with at least one neutralizer or neutralizing component.

In embodiments, an entirety of an external surface of the foam element is covered or draped by a layer of a sheet material. In embodiments, an external surface of the foam element is covered or draped partially by a sheet material. In embodiments, the foam element is contained within a volume defined by walls made of the sheet material. The sheet material is configured to allow moisture to enter from the outside and complementary material to exit. In other embodiments, no portion of an external surface of the foam element is covered or draped by a layer of a sheet material. Suitable sheet materials are capable of transmitting moisture and may e.g. be made from polymers such as polyolefin types e.g. polyethylene, polypropylene or polybutylene, polyamide such as nylon, polyurethane, polyvinyl acetate, polyvinyl chloride, fluorinated polyvinyl compound, polyvinylidene chloride, polyvinyl alcohol, ethylene vinyl acetate, cellulose acetate or other thermoplastic polysaccharides, polyether block amides like PEBAX , block copolymers like styrene-isoprene-styrene block copolymers or ethylene acrylate block copolymers, polyesters such as polyethylene terephthalate (PET) or derivates thereof and any laminates from such polymers. The sheet material can suitably be provided as a "cover" of a relatively thin foam layer like made from polyurethane, polyethylene or polyvinyl acetate.

In embodiments, the foam element is made from a soft foam. In embodiments, softness can be defined by the perception of a polyester foam with a PPI (Pore per Inch) of 75-85, and a CLD (Compression Load Deflection) of 0.20 psi at 25% and a CLD of 0.4 psi at 65%.

In embodiments, the foam element comprises a hydrophilic foam. In embodiments, the foam element comprises a polyurethane foam such as, but not limited to, a polyester polyurethane foam.

In embodiments, the complementary material dispenser is configured as a bulk mass of complementary material comprising an adhesive material component. The adhesive material component provides for the bulk mass of the complementary material dispenser to be attachable to an interior surface of a top portion of a collecting bag for body waste. In embodiments, the bulk mass of the dispenser is provided on the distal wall of the collecting bag across from a body waste inlet opening in the proximal wall of the collecting bag. By providing the complementary material dispenser as a bulk mass, the body waste or output can be immediately exposed to the complementary material. Moreover, the bulk mass provides production friendly embodiments of the complementary material dispenser. In embodiments, the bulk mass of the complementary material can include one or more different material compositions. In embodiments, the bulk mass of the complementary material includes at least one neutralizer material. In embodiments, the bulk mass of the complementary material includes at least one hydrocolloid. Including a hydrocolloid can help to absorb excessive amounts of moisture in the bag and thereby help to increase the 'lifetime' of the bulk mass complementary material dispenser. In should be understood, that the phrase 'bulk mass' means that these embodiments are not covered or draped by a sheet material, nor is the complementary material contained in a receptacle.

In embodiments, the complementary material dispenser is configured to be manually attachable to the interior surface of the top portion of the collecting bag. Thereby, the complementary material dispenser of the disclosure allows for a user or health care professional to attach it to the collecting bag when the user's waste collecting appliance is changed. These embodiments can further be advantageous in that the complementary material dispenser of the disclosure can be applied and useful in combination with different types, sizes and brands of body waste collecting bags.

The complementary material dispenser includes attachment means on an exterior surface of the dispenser for attachment of the dispenser to the interior surface of the top portion of the collecting bag. In embodiments, the attachment means can be of any suitable kind, such as, but not limited to, hook and loop-type means (Velcro^{®}), adhesive surfaces and/or flanges and wire/lace connections.

In embodiments, the complementary material dispenser includes attachment means provided at or along a top end portion of an exterior surface of the dispenser. In embodiments, the attachment means is a piece of material comprising one half of a Velcro^{®} connection. In one embodiment, the piece of material comprises a circular shape, providing a 'Velcro^{®}-dot'. In one embodiment, the piece of material comprises a linear shape. In embodiments, the dispenser includes two or more pieces of material comprises one half of a Velcro^{®} connection.

In embodiments, the complementary material dispenser includes a pocket on an exterior surface of the dispenser, the pocket configured for engagement with a finger of a user. Particularly, the pocket is dimensioned to accommodate one finger of an average person. The pocket facilitates manual attachment of the dispenser to an interior surface of the top portion of a body waste collecting bag.

In embodiments, the complementary material of the complementary material dispenser includes one or more neutralizers or neutralizing components.

### The neutralizer (neutralizing component)

By neutralizer (neutralizing component) is herein meant a neutralizing substance capable of neutralizing or at least minimizing the level of skin- or adhesive- aggressiveness of body waste, such as stomal output.

In embodiments, the neutralizer comprises a clay, such as organophilic clay, for example bentonite or synthetic clay such as laponite. Examples of such clays are disclosed in EP 1 140 009.

In embodiments, the neutralizer comprises potato-derived inhibitors or protease inhibitors. Examples of potato-derived inhibitors such as potato protein are disclosed in EP 1 736 136.

In embodiments, the neutralizing component can include an adhesive. In other embodiments, the neutralizing component comprises a powder. In other embodiments, the neutralizing component comprises a liquid. In other embodiments, the neutralizing component comprises a gel. In other embodiments, the neutralizing component comprises a plurality of pellets. In yet other embodiments, the neutralizing component comprises a combination of any one or more of an adhesive, a powder, a liquid, a gel and/or a plurality of pellets. These options each provides one or more different advantages such as including, but not limited to, manipulability, shelf life, suitability for different kinds of stomal output (colostomy output tends to be much more solid than ileo- and urostomy output), processing characteristics during manufacture and others. By selectively applying these options, individually or in combination, to meet requirements of a target ostomy/ostomist group, the suitability of the ostomy appliance and the improvement in reduction or elimination of the problems discussed above, including reducing the risk of leakage, can be significantly improved.

Particularly, in embodiments wherein the neutralizing component comprises an adhesive, suitable materials include adhesives, such as, but not limited to, adhesive pastes. Suitable materials for a paste-type adhesive comprise adhesives of the types disclosed in WO2010/069334. Other types of adhesive pastes are also acceptable.

In embodiments, the complementary material can include more than one neutralizing component. In embodiments, each neutralizing component can include more than one kind of neutralizer (e.g. directed towards neutralizing different substances of the stomal output). In some implementations, the helpful effect(s) presented by one neutralizing component and/or one neutralizer can be amplified by the presence of another kind of neutralizing component and/or neutralizer to provide even better results in terms of preventing or at least reducing the prevalence of adhesive failures, skin damage and/or maceration, leakage incidents etc. This in turn helps provide for longer wear time of the ostomy appliance.

### Ostomy appliance system

The disclosure relates to an ostomy appliance system comprising a complementary material dispenser as disclosed herein, a body waste collecting bag and a base plate being attachable to the body waste collecting bag.

The body waste collecting bag includes a proximal wall and a distal wall, the walls combining to form an interior surface of the collecting bag. The proximal wall includes an inlet opening. The inlet opening is configured to receive the stoma and/or stomal output from a user.

The base plate includes an adhesive attachable to the skin surface of a user around a stoma of the user. In embodiments, the base plate comprises an adhesive provided on a first surface of a carrier film. In embodiments, the base plate comprises a release liner provided on the adhesive first surface of the carrier film.

The complementary material dispenser is configured to be attachable to the interior surface of a top portion of the collecting bag, such that complementary material being dispensed from the dispenser is guided towards a material allocation zone at the top portion of the collecting bag.

In embodiments, the complementary material dispenser is attached to the interior surface of the top portion of the collecting bag at a top peripheral weld zone attaching the proximal wall of the collecting bag to the distal wall of the collecting bag. This provides embodiments, wherein the complementary material dispenser is attached to the collecting bag of the ostomy appliance system already at manufacture. Substitution of the complementary material dispenser is then effectuated each time the user exchanges the used ostomy appliance for a new one.

In a comparative example, the complementary material dispenser is attachable to the base plate of the ostomy appliance by a user or a health care professional. Such examples *inter alia* allow for easier adaptation of a stoma-receiving opening in the base plate when customization of the size and shape of the opening to the user's stoma is required, because the dispenser does not interfere with the cutting or adaptation. Alternatively, in examples as can be seen from the above, the dispenser is attached to the base plate at manufacture and delivered to the user in an attached configuration.

In embodiments, the collecting bag is already attached to the base plate at manufacture and may be understood as a one-piece ostomy appliance, as is commonly understood in the ostomy appliance area. Such embodiments of a one-piece ostomy appliance of the disclosure provide one-piece appliances which may have prolonged wear time, due to the combination with the attached or attachable complementary material dispenser.

The complementary material dispenser includes attachment means. The attachment means are provided on an exterior surface of the dispenser. The attachment means is/are configured to for attachment of the dispenser to the interior surface of the top portion of the collecting bag.

In embodiments, the collecting bag and the complementary material dispenser includes attachment means provided on each of the collecting bag and the complementary material dispenser. In embodiments, the attachment means provided on the complementary material dispenser is/are configured to attach or connect with the attachment means provided on the collecting bag. In embodiments, the attachment means comprise pairs of means of the hook-and-loop type, such as a Velcro^{®} connection.

In embodiments, a first coupling half is provided on the base plate. The body waste collecting bag includes a second coupling half provided around an inlet opening in the proximal wall of the collecting bag. This provides for the complementary material dispenser of the disclosure to also be used in an ostomy appliance system which improves the life- or wear time also of a two-piece ostomy appliance, also a phrase commonly understood in the area.

In embodiments, the first coupling half is a flange adapted to provide a surface for attaching another coupling half in the form of an adhesive flange provided on the collecting bag. In embodiments, the first coupling half is configured as a flexible, planar annular flange optionally comprising an adhesive. The first coupling half is adapted to couple with the second coupling half provided around an inlet opening of the collecting bag by means of an adhesive. The adhesive coupling may provide a releasable or a permanent adhesive coupling engagement.

In embodiments, the first coupling half is an annular ring comprising an upstanding flange protruding from the distal surface perpendicular thereto for attaching a second coupling half in the form of a coupling ring provided around an inlet opening of the collecting bag.

In embodiments, the ostomy appliance system is provided as a kit of parts including a packaging configured to contain at least one complementary material dispenser, at least one base plate for an ostomy appliance and at least one collecting bag adapted to be attached to the base plate. The complementary material dispenser is attachable to the base plate as described in this disclosure. In embodiments, the kit of parts includes a set of instructions for use of the combination of the components of the ostomy appliance system, and particularly with instructions on how to apply and use the complementary material element in combination with the base plate and the collecting bag of the ostomy appliance system.

### Detailed description of the drawings

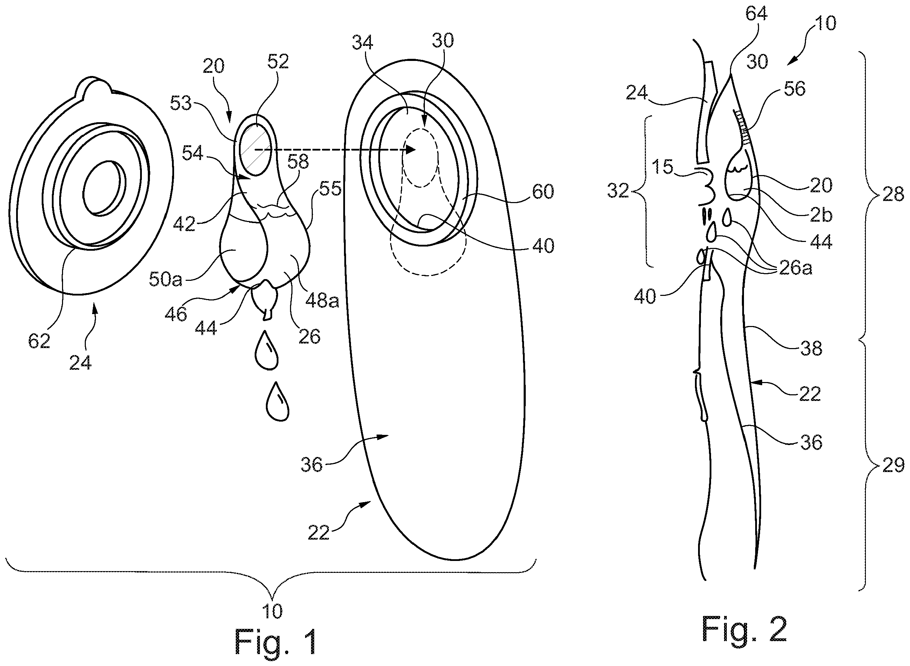

Figure 1 is a schematic perspective view of one embodiment of a complementary material dispenser 20 attachable to an interior surface 30 of a top portion 32 of a collecting bag 22 for body waste. The complementary material dispenser 20 is configured to provide dispensing of complementary material 26 from the dispenser 20 towards a material allocation zone 32 at the top portion 28 of the collecting bag 22.

In the embodiment of Figure 1, the material allocation zone 32 is defined partly at the body waste inlet opening 34 of the collecting bag 22 and partly at the interior surface of a proximal wall 36 of the collecting bag 22 adjacent to and at a bottom-most portion 40 of the body waste inlet opening 34. Figure 1 further illustrates how embodiments of the dispenser 20 directs complementary material 26 (illustrated as drops 26a) to the material allocation zone 32 at the bottom-most portion 40 of the opening 34.

In the embodiment of Figure 1, the complementary material dispenser 20 is a receptacle 42 for housing the complementary material 26. The receptacle 42 contains the releasable complementary material 26. In the illustrated embodiment, the receptacle 42 takes the form of a bag or pouch containing the complementary material 26.

The receptacle 26 includes one outlet 44 for dispensing the complementary material 26 from the dispenser 20. In Figure 1, the receptacle 42 includes the one outlet 44 provided in a first end portion 46 of the receptacle 42. The first end portion 46 is configured to be located closest to a bottom portion 29 of a collecting bag 22, when the dispenser 20 is attached to the interior surface 30 of the top portion 28 of the collecting bag 22 (Figure 2). In the embodiments of Figures 1 and 2, the receptacle 42 includes two major external surfaces 48a, 48b and two minor external surfaces 50a, 50b (of which only surfaces 48a, 50a are visible), wherein the one outlet 44 provided along an edge where the first and second major external surfaces 48a, 48b are connected to each other. The two major external surfaces 48a, 48b extend substantially in parallel with the proximal wall 36 and the distal wall 38 of the collecting bag 22. The two minor external surfaces 50a, 50b combine with the two major external surfaces 48a, 48b to form sides of the receptacle 42.

Figures 1 and 2 further illustrate how the one outlet 44 of the receptacle 42 helps provide and control the delivering of the complementary material 26 from the dispenser 20 towards the material allocation zone 32, including directing the complementary material 26 towards a location or area of the ostomy appliance around the stoma 15.

Figure 1 further illustrates the complementary material dispenser 20 including attachment means 52. The attachment means 52 is provided on an exterior surface 54 of the dispenser 20. The attachment means 52 configures the dispenser 20 for attachment to the interior surface 30 of the top portion 28 of the collecting bag 22. In Figure 1, the attachment means 52 comprises means of the hook-and-loop type in the form of a Velcro^{®} connection. In embodiments, as also illustrated by Figures 1 and 2, the attachment means 52 forms one half of a connection and is adapted to attach to a corresponding second half attachment means 54 on the interior surface 30 of the top portion 28 of the collecting bag 22. In embodiments as illustrated in Figures 1 and 2, the attachment means 52 and/or attachment means 56 is/are glued, adhered or welded to the exterior surface 54 of the dispenser 20, and to the interior surface 30 of the collecting bag 22, respectively. In embodiments as illustrated in Figure 1, the receptacle 42 of the dispenser 20 comprises a flange portion 53 extending from a reservoir portion 55 of the receptacle 42 to which flange portion 53 the attachment means 52 is attached.

In the illustration of Figure 1, the complementary material 26 is illustrated as visible at position 58 through the major external surface 48a and the minor external surface 50a. However, it is to be understood that the external surfaces 48a, 48b, 50a, 50b can also be opaque and/or include an opaque sheet material partially or entirely covering the dispenser 20.

The schematic Figure 1 further illustrates an ostomy appliance system 10 including a base plate 24 for attaching (adhering) the appliance to the user's skin around a stoma 15 (Figure 2), a collecting bag 22 for collecting the body waste or stomal output and a complementary material dispenser 20 attachable to an interior surface 30 of a top portion 28 (Figure 2) of the collecting bag 22 (as indicated by the dotted lines in Figure 1). The collecting bag 22 is attachable to the base plate 24 by coupling means 60 and 62 provided on the bag 22 and base plate 24 respectively.

Figure 2 is a schematic cross-sectional view of the ostomy appliance system 10 including a complementary material dispenser 20 attached to an interior surface 30 of the top portion 28 of the collecting bag 22.

In embodiments (not shown), the complementary material dispenser 20 is attached to the interior surface 30 of the top portion 28 of the collecting bag 22 at a peripheral weld zone 64 at the top portion 28 of the bag 22, where the proximal wall 36 of the collecting bag 22 is attached to the distal wall 38 of the collecting bag 22.

Figure 2 further illustrates one application of the dispenser 20 being located in the collecting bag 22, such that the complementary material 26 (in Figure 2 additionally illustrated as drops of material 26a) being dispensed from the dispenser 20 is guided towards a material allocation zone 32 at the top portion 28 of the collecting bag 22. In this regard, it is noted that the illustration of Figure 2 should be understood as a somewhat idealistic view of the real-life situation in which the ostomy appliance system 10 is used - meaning that, when being worn by the user during normal use, clothing and other external influences act on the distal wall 38 of the collecting bag 22 to push it much closer and into contact with the proximal wall 36, as well as into contact with the whole peristomal area. This in turn means that the dispenser 20 is also closer to the stoma 15 and a distal surface of the base plate 24, whereby delivery of complementary material 26 being dispensed from the dispenser 20 is easily guided by gravity towards or to the material allocation zone 32. It is further understood that in comparative examples, the dispenser 20 can locate above or to the sides of the stoma 15 inside the collecting volume of the bag 22, i.e. in an alternative manner in comparison to what is illustrated in Figure 2.

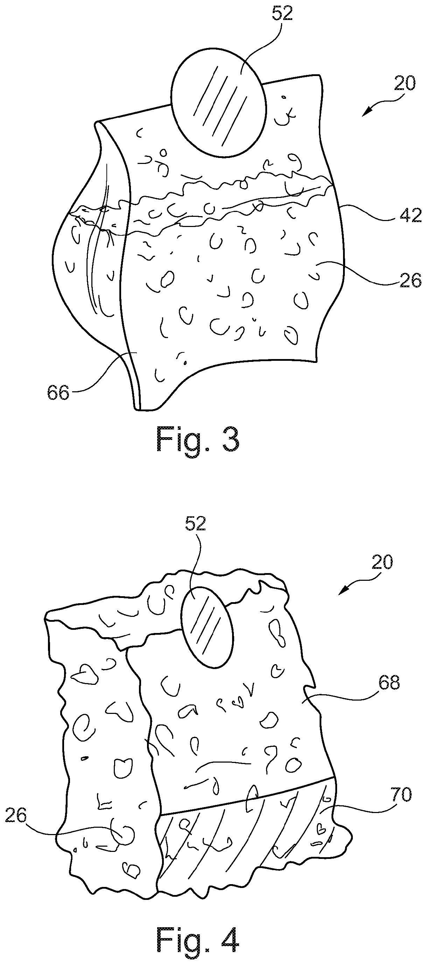

Figure 3 is a schematic perspective view of one embodiment of a complementary material dispenser 20. In the embodiment of Figure 3, the receptacle 42 is provided in the form a 'tea bag' containing the complementary material 26. The dispenser 20 includes attachment means 52 for attaching the dispenser 20 to the interior surface of a collecting bag. In embodiments as illustrated by the dispenser 20 of Figure 3, the receptacle 42 can include walls 66 made from a moisture-penetrable material allowing moisture to enter from outside to inside the receptacle 42, and complementary material 26 to be dispensed from inside the receptacle 42 to the outside thereof. Additionally, the 'tea bag' receptacle 42 of Figure 3 can comprise one or more outlets (not shown) for dispensing the complementary material 26.

Figure 4 is a schematic perspective view of one embodiment of a complementary material dispenser 20. In the embodiment of Figure 4, the receptacle 42 comprises a foam element 68 infused with complementary material 26. The dispenser 20 includes attachment means 52 for attaching the dispenser 20 to the interior surface of a collecting bag. In embodiments as illustrated by the dispenser 20 of Figure 3, the foam element 68 allows moisture to enter the element 68 to release the infused complementary material 26 from the foam structure and to dispense the material 26 to the surroundings, particularly to a material allocation zone at a top portion of a collecting bag. In Figure 4, an external surface of the foam element 68 is covered or draped partially by a sheet material 70. The sheet material 70 is configured to allow moisture to enter from the outside and complementary material 26 to exit the dispenser 20.

Although specific embodiments have been illustrated and described herein, it will be appreciated by those of ordinary skill in the art that a variety of alternate and/or equivalent implementations may be substituted for the specific embodiments shown and described without departing from the scope of the present invention. This application is intended to cover any adaptations or variations of body side members for ostomy appliances as discussed herein. Therefore, it is intended that this invention be limited only by the claims.

## Claims

1. An ostomy appliance system (10), comprising:
a body waste collecting bag (22) comprising a proximal wall (36) and a distal wall (38), the walls combining to form an interior surface (30) of the collecting bag and the proximal wall (36) further comprising an inlet opening (34);
a base plate (24) comprising an adhesive attachable to the skin surface of a user around a stoma of the user, the base plate (24) further being attachable to the collecting bag (22), and
a complementary material dispenser (20) configured to be attachable to an interior surface (30) of a top portion (28) of the collecting bag (22), and configured to provide dispensing of complementary material (26) from the dispenser (20) towards a material allocation zone (32) at the top portion (28) of the collecting bag,
wherein the dispenser (20) comprises attachment means (52) on an exterior surface (54) of the dispenser (20) for attachment of the dispenser to the interior surface (30) of the top portion (28) of the collecting bag, **characterized in that** the complementary material (26) is suitable to neutralize the aggressive contents of the body waste to help avoid damage to the skin of the user and degradation of the adhesive of the base plate (24).

2. The system of claim 1, wherein the complementary material dispenser (20) comprises a receptacle (42) for housing the complementary material (26).

3. The system of claim 2, wherein the receptacle (42) is configured to be open, closed or partially opened.

4. The system of claim 2, wherein the receptacle (42) is configured to be openable.

5. The system of claim 2, wherein the receptacle (42) comprises at least one outlet for dispensing the complementary material (26).

6. The system of claim 1, wherein the complementary material dispenser (20) comprises a foam element (68).

7. The system of claim 6, wherein one or more material compositions is/are contained in the foam element (68).

8. The system of claim 6, wherein the foam element (68) is infused with one or more material compositions.

9. The system of any one of claims 6-8, wherein at least one neutralizer or neutralizing component is contained in the foam element (68).

10. The system of any one of claims 6-8, wherein the foam element (68) is infused with at least one neutralizer or neutralizing component.

11. The system of any one of claims 6-10, wherein an entirety of an external surface of the foam element (68) is covered or draped by a layer of a sheet material.

12. The system of any one of claims 6-10, wherein an external surface of the foam element (68) is covered or draped partially by a sheet material.

13. The system of claim 1, wherein the complementary material dispenser(20) is configured as a bulk mass of complementary material comprising an adhesive material.

14. The system of claim 1, wherein the dispenser (20) is configured to be manually attachable to the interior surface (30) of the top portion (28) of the collecting bag (22).

15. The system of claim 14, wherein the dispenser (20) comprises a pocket on an exterior surface of the dispenser, the pocket configured for engagement with a finger of a user such as to facilitate manual attachment of the dispenser to the interior surface (30) of the top portion (28) of the collecting bag (22).

16. The system of any preceding claim, wherein the complementary material (26) comprises one or more neutralizers.

17. The ostomy appliance system of claim 1, wherein the complementary material dispenser (20) is attached to the interior surface (30) of the top portion (28) of the collecting bag (22) at a peripheral weld zone (62) where the proximal wall (36) of the collecting bag is attached to the distal wall (38) of the collecting bag.

## Patentansprüche

1. Ostomievorrichtungssystem (10), umfassend:
einen Sammelbeutel (22) für Körperausscheidungen, umfassend eine proximale Wand (36) und eine distale Wand (38), wobei die Wände unter Bildung einer inneren Oberfläche (30) des Sammelbeutels kombinieren und die proximale Wand (36) des Weiteren eine Einlassöffnung (34) umfasst;
eine Basisplatte (24), die einen Klebstoff umfasst, der an der Hautoberfläche eines Benutzers um ein Stoma des Benutzers herum befestigbar ist, wobei die Basisplatte (24) des Weiteren an dem Sammelbeutel (22) befestigbar ist, und
einen Komplementärmaterialspender (20), der ausgestaltet ist, um an der inneren Oberfläche (30) eines oberen Anteils (28) des Sammelbeutels (22) befestigbar zu sein, und ausgestaltet ist, um Spenden von Komplementärmaterial (26) aus dem Spender (20) in Richtung einer Materialzuteilungszone (32) an dem oberen Anteil (28) des Sammelbeutels bereitzustellen,
wobei der Spender (20) Befestigungsmittel (52) auf einer äußeren Oberfläche (54) des Spenders (20) zur Befestigung des Spenders an der inneren Oberfläche (30) des oberen Anteils (28) des Sammelbeutels umfasst,
**dadurch gekennzeichnet, dass** das Komplementärmaterial (26) zum Neutralisieren der aggressiven Inhalte der Körperausscheidung geeignet ist, um dazu beizutragen, Schäden an der Haut des Benutzers und Abbau des Klebstoffs der Basisplatte (24) zu vermeiden.

2. System nach Anspruch 1, wobei der Komplementärmaterialspender (20) einen Behälter (42) zum Aufnehmen des Komplementärmaterials (26) umfasst.

3. System nach Anspruch 2, wobei der Behälter (42) ausgestaltet ist, um offen, geschlossen oder teilweise geöffnet zu sein.

4. System nach Anspruch 2, wobei der Behälter (42) ausgestaltet ist, um öffnungsfähig zu sein.

5. System nach Anspruch 2, wobei der Behälter (42) mindestens einen Auslass zum Spenden des Komplementärmaterials (26) umfasst.

6. System nach Anspruch 1, wobei der Komplementärmaterialspender (20) ein Schaumelement (68) umfasst.

7. System nach Anspruch 6, wobei eine oder mehrere Materialzusammensetzungen in dem Schaummaterial (68) enthalten ist bzw. sind.

8. System nach Anspruch 6, wobei das Schaumelement (68) mit einer oder mehreren Materialzusammensetzungen infundiert ist.

9. System nach einem der Ansprüche 6 bis 8, wobei mindestens ein Neutralisierungsmittel oder eine Neutralisierungskomponente in dem Schaumelement (68) enthalten ist.

10. System nach einem der Ansprüche 6 bis 8, wobei das Schaumelement (68) mit mindestens einem Neutralisierungsmittel oder einer Neutralisierungskomponente infundiert ist.

11. System nach einem der Ansprüche 6 bis 10, wobei eine Gesamtheit einer externen Oberfläche des Schaumelements (68) von einer Schicht eines Folienmaterials bedeckt oder abgedeckt ist.

12. System nach einem der Ansprüche 6 bis 10, wobei eine externe Oberfläche des Schaumelements (68) teilweise von einem Folienmaterial bedeckt oder abgedeckt ist.

13. System nach Anspruch 1, wobei der Komplementärmaterialspender (20) als Hauptteil der Masse an Komplementärmaterial ausgestaltet ist, das ein Klebstoffmaterial umfasst.

14. System nach Anspruch 1, wobei der Spender (20) ausgestaltet ist, um manuell an der inneren Oberfläche (30) des oberen Anteils (28) des Sammelbeutels (22) befestigbar zu sein.

15. System nach Anspruch 14, wobei der Spender (20) eine Tasche an einer äußeren Oberfläche des Spenders umfasst, wobei die Tasche zum Eingriff mit einem Finger eines Benutzers ausgestaltet ist, um so manuelle Befestigung des Spenders an der inneren Oberfläche (30) des oberen Anteils (28) des Sammelbeutels (22) zu erleichtern.

16. System nach einem der vorhergehenden Ansprüche, wobei das Komplementärmaterial (26) ein oder mehrere Neutralisierungsmittel umfasst.

17. Ostomievorrichtungssystem nach Anspruch 1, wobei der Komplementärmaterialspender (20) an der inneren Oberfläche (30) des oberen Anteils (28) des Sammelbeutels (22) an einer peripheren Schweißzone (62) befestigt ist, wo die proximale Wand (36) des Sammelbeutels an der distalen Wand (38) des Sammelbeutels befestigt ist.

## Revendications

1. Système d'appareil d'ostomie (10), comprenant :
un sac de collecte de déchets corporels (22) comprenant une paroi proximale (36) et une paroi distale (38), les parois se combinant pour former une surface intérieure (30) du sac de collecte et la paroi proximale (36) comprenant en outre une ouverture d'entrée (34) ;
une plaque de base (24) comprenant un adhésif pouvant être fixé à la surface de la peau d'un utilisateur autour d'une stomate de l'utilisateur, la plaque de base (24) pouvant en outre être fixée au sac de collecte (22), et
un distributeur de matériau complémentaire (20) configuré pour pouvoir être fixé à une surface intérieure (30) d'une partie supérieure (28) du sac de collecte (22), et configuré pour fournir une distribution de matériau complémentaire (26) à partir du distributeur (20) vers une zone d'attribution de matériau (32) au niveau de la partie supérieure (28) du sac de collecte,
le distributeur (20) comprenant des moyens de fixation (52) sur une surface extérieure (54) du distributeur (20) pour la fixation du distributeur à la surface intérieure (30) de la partie supérieure (28) du sac de collecte, **caractérisé en ce que** le matériau complémentaire (26) est approprié pour neutraliser le contenu agressif des déchets corporels pour aider à éviter les dommages à la peau de l'utilisateur et la dégradation de l'adhésif de la plaque de base (24).

2. Système selon la revendication 1, le distributeur de matériau complémentaire (20) comprenant un réceptacle (42) pour loger le matériau complémentaire (26).

3. Système selon la revendication 2, le réceptacle (42) étant configuré pour être ouvert, fermé ou partiellement ouvert.

4. Système selon la revendication 2, le réceptacle (42) étant configuré pour pouvoir être ouvert.

5. Système selon la revendication 2, le réceptacle (42) comprenant au moins une sortie pour distribuer le matériau complémentaire (26).

6. Système selon la revendication 1, le distributeur de matériau complémentaire (20) comprenant un élément en mousse (68).

7. Système selon la revendication 6, une ou plusieurs compositions de matériaux étant contenues dans l'élément en mousse (68).

8. Système selon la revendication 6, l'élément en mousse (68) étant infusé avec une ou plusieurs compositions de matériaux.

9. Système selon l'une quelconque des revendications 6 à 8, au moins un neutralisant ou un composant de neutralisation étant contenu dans l'élément en mousse (68).

10. Système selon l'une quelconque des revendications 6 à 8, l'élément en mousse (68) étant infusé avec au moins un neutralisant ou un composant de neutralisation.

11. Système selon l'une quelconque des revendications 6 à 10, une totalité d'une surface externe de l'élément en mousse (68) étant couverte ou enveloppée par une couche d'un matériau en feuille.

12. Système selon l'une quelconque des revendications 6 à 10, une surface externe de l'élément en mousse (68) étant recouverte ou enveloppée partiellement par un matériau en feuille.

13. Système selon la revendication 1, le distributeur de matériau complémentaire(20) étant configuré comme une masse brute de matériau complémentaire comprenant un matériau adhésif.

14. Système selon la revendication 1, le distributeur (20) étant configuré pour pouvoir être fixé manuellement à la surface intérieure (30) de la partie supérieure (28) du sac de collecte (22).

15. Système selon la revendication 14, le distributeur (20) comprenant une poche sur une surface extérieure du distributeur, la poche étant configurée pour s'engager avec un doigt d'un utilisateur de manière à faciliter la fixation manuelle du distributeur à la surface intérieure (30) de la partie supérieure (28) du sac de collecte (22).

16. Système selon n'importe quelle revendication précédente, le matériau complémentaire (26) comprenant un ou plusieurs neutralisants.

17. Système d'appareil d'ostomie selon la revendication 1, le distributeur de matériau complémentaire (20) étant fixé à la surface intérieure (30) de la partie supérieure (28) du sac de collecte (22) au niveau d'une zone de soudure périphérique (62) où la paroi proximale (36) du sac de collecte est fixée à la paroi distale (38) du sac de collecte.
